# EUROPEAN PATENT SPECIFICATION

(11) **EP 0 662 819 B1**
(45) Date of publication and mention of the grant of the patent: **24.04.1996**
(21) Application number: 93919042.7
(22) Date of filing: 09.09.1993
(51) Int. Cl.: A61K 9/107, A61K 31/52

(54) **AN ANTIVIRALLY ACTIVE PHARMACEUTICAL OIL-IN-WATER EMULSION CONTAINING 9-[(2-HYDROXYETHOXY)METHYL]GUANINE (ACYCLOVIR) OR A SALT OR ESTER THEREOF**
ANTIVIRAL WIRKSAME PHARMAZEUTISCHE ÖL IN WASSER-EMULSION, DIE 9-[(2-HYDROXYETHOXY)METHYL]-GUANIN (ACYCLOVIR) ODER EIN SALZ ODER EINEN ESTER DAVON ENTHÄLT
EMULSION PHARMACEUTIQUE D'HUILE DANS L'EAU A ACTION ANTIVIRALE CONTENANT DE LA 9-[(2-HYDROXYETHOXY)METHYL]GUANINE (ACYCLOVIR) OU UN SEL OU ESTER DE LADITE SUBSTANCE

(30) Priority: 09.09.1992 DK 1113/92
(43) Date of publication of application: 19.07.1995
(73) Proprietor: A/S GEA Farmaceutisk Fabrik, DK-2000 Frederiksberg (DK)
(72) Inventor: GEBHARD-HANSEN, Knud Erik, DK-3460 Birkerod (DK); PEDERSEN, Soren Bols, DK-2650 Hvidovre (DK); BJORNSDOTTIR, Karen, DK-3500 Vaerlose (DK); LISSAU, Bodil Gyllembourg, DK-2000 Frederiksberg (DK); ALHEDE, Borge Ingvar Frisch, DK-2670 Greve Strand (DK)
(74) Representative: Mathiesen, Hans Preben
(86) International application number: DK9300288
(87) International publication number: WO9405258

(56) References cited:
- EP-A- 0 044 543

## Description

### Technical Field

The present invention relates to an antivirally active pharmaceutical oil-in-water emulsion containing 9-[(2-hydroxyethoxy)methyl]guanine (acyclovir) or a salt or ester thereof as an active ingredient in the continuous aqueous phase, said phase in addition to said active ingredient and the dispersed oil phase containing a water miscible organic solvent, wherein a polyhydric alcohol may form a constituent.

### Background Art

GB patent No. 1.523.865 discloses that acyclovir and pharmaceutically acceptable salts and esters thereof have an antiviral activity against various classes of DNA and RNA viruses, both in vitro and in vivo. In particular, the compound is active against herpes simplex virus which causes herpetic keratitis in rabbits, herpetic encephalitis in mice and cutaneous herpes in guinea pigs.

Acyclovir has a low solubility in water and is almost totally insoluble in hydrophobic solvent systems, which is why it is difficult to produce a topical formulation containing such a high concentration of dissolved acyclovir enabling it to exert its full effect. It is thus difficult to obtain an optimum penetration of said compound into the skin.

In addition to a sufficient concentration of pharmaceutically active compound, which inter alia depends on a sufficient rate of dissolution of the active compound in the chosen solvent, it is important that formulations containing the pharmaceutically active compound are stable and thus do not lose their potency during storage for long periods of time or discolour or are unduly irritating to skin or mucosa after having been applied.

Example 26 of said GB patent relates to an oil-in-water cream containing 5% w/w acyclovir and 5% w/w propylene glycol. In this example the function of the propylene glycol is to act as an humectant, i.e. as a hydroscopic ingredient, which improves the cosmetic sensation by use of the cream and further limits dehydration during storage. Animal tests with this cream and another aqueous cream B.P. (British Pharmacopoeia) containing acyclovir did not provide a particularly rapid cure, probably due to an insufficient amount of dissolved active ingredient and consequently poor penetration of acyclovir into the skin.

Because of the lipid nature of the skin surface and especially the stratum corneum, it has long been thought that in order to achieve a good transdermal penetration into the skin the active ingredient in an emulsion should be located in the oil phase so that it may spread into the lipid components of the skin.

However, it has been found that in order to obtain optimum release of acyclovir from topical formulations, the external phase and thus the aqueous phase of an oil-in-water emulsion should contain the maximum concentration of solubilised drug. EP-A1-0 044 543 thus discloses that by using a greater concentration of polyhydric alcohol than the 5% w/w as known from said GB patent, i.e. at least 50% v/v of the aqueous phase, an increased concentration of solubilised acyclovir and thus an enhanced activity and efficacy of the emulsion formulation may be obtained.

Topical acyclovir formulations containing such an increased concentration of polyhydric alcohol as co-solvent in the aqueous phase have been found to have the adequate stability and are not unduly irritating to the skin or mucosa. Compared to the prior art formulation containing 5% w/w acyclovir and only 5% w/w propylene glycol the formulations known from EP-A1-0 044 543 penetrate the skin more effectively and in a greater concentration of acyclovir, whereby a more rapid cure of the infection concerned is obtained. These advantages are obtained when the formulation comprises from 1% to 10% w/w of acyclovir or a salt or ester thereof, from 30% to 50 % w/w of the polyhydric alcohol and from 20% to 40% w/w water, said percentages being based on the total weight of the formulation.

### Disclosure of the Invention

It has now surprisingly been found that a substantially increased concentration of dissolved acyclovir and thus an enhanced penetration of acyclovir into the skin may be obtained by using an oil-in-water emulsion of the type dealt with by using glycerol formal as organic solvent in the continuously aqueous phase instead of a polyhydric alcohol or by replacing part of the polyhydric alcohol by glycerol formal, and the antivirally active pharmaceutical oil-in-water emulsion according to the invention is thus characterised in that the emulsion comprises from 1% to 10% w/w of acyclovir or a salt or ester thereof, from 20% to 50 % w/w of organic solvent comprising from 5% to 50% w/w glycerol formal and from 0% to 29% w/w of a polyhydric alcohol, and from 20% to 40% w/w water, said percentages being based on the total weight of the formulation.

According to a preferred embodiment of the emulsion of the invention the emulsion comprises from 2% to 5% w/w acyclovir or a salt or ester thereof, from 30% to 45% w/w solvent, and from 15% to 35% w/w water, more preferred 5% w/w acyclovir or a salt or ester thereof, 40% w/w glycerol formal, and from 15% to 30% w/w water.

It has been found that the emulsion according to the present invention results in an substantially enhanced penetration of the active ingredient, acyclovir, into the skin, the enhanced effect being due to the use of glycerol formal as organic solvent in the emulsion or as a considerable constituent thereof.

The enhanced solubility of acyclovir obtained by using glycerol formal instead of propylene glycol or as substitute for a part of the propylene glycol contents in an oil-in-water emulsion containing a water miscible organic solvent appears from the following solubility test.

### Solubility of acyclovir in glycerol formal/water mixtures and in glycerol formal/propylene glycol/water mixtures.

A surplus of acyclovir is added to glycerol formal/water mixtures and glycerol formal/propylene glycol/water mixtures at room temperature, and the mixtures are then stirred for 2 hours and filtered. Subsequent to adequate dilution with 0.1 N NaOH the solutions are spectrophotometrically measured at 260 nm against 0.1 N NaOH. A 0.01% solution of acyclovir in 0.1 N NaOH is used as standard.

**Table I**

| Solubility of acyclovir in mixtures of glycerol formal and water: | |
|---|---|
| Percentage by volume of glycerol formal in water | Solubility of acyclovir in mg/ml |
| 0 | 1.17 |
| 10 | 1.63 |
| 25 | 2.64 |
| 50 | 4.37 |
| 60 | 4.67 |
| 75 | 4.15 |
| 90 | 2.97 |
| 100 | 3.27 |

It appears from table I that acyclovir surprisingly is more soluble in mixtures of glycerol formal and water than in glycerol formal and water, respectively, and that the maximum solubility is obtained at a ratio of approximately 60 v/v glycerol formal and 40 v/v water.

**Table II**

| Solubility of acyclovir in mixtures of glycerol formal, propylene glycol, and water: | |
|---|---|
| Glycerol formal/propylene glycol/water g/g/ml | Solubility of acyclovir in mg/ml |
| 0/40/20 | 3.44 |
| 11/29/20 | 4.71 |
| 20/20/20 | 4.60 |
| 30/10/20 | 5.20 |
| 40/0/20 | 5.73 |

It appears from table II that acyclovir is dissolved increasingly, when propylene glycol is replaced by glycerol formal in mixtures of glycerol formal, propylene glycol and water.

### Skin penetration tests

A number of skin penetration tests have been carried out in vitro with human skin to show the enhanced skin penetration by use of oil-in-water emulsions according to the invention compared to the penetration obtained by using the emulsion formulation known from DK patent No. 149.191.

For screening of emulsion formulations in the form of creams with different compositions so-called Franz Diffusion Cells were used consisting of glass chambers surrounded by a thermostatically controlled water jacket. Pieces of skin (from plastic surgery operations) are fixed on top of the chambers with stratum corneum facing upwards and a physiological fluid (receiver fluid) is filled into the chambers. The cream to be examined is applied to the skin and at suitable intervals samples of the receiver fluid are taken for analysis of the active substance (acyclovir). In order to minimize the variation between the barrier properties of individual pieces of skin, a measurement of the water permeability of each piece of skin is carried out to begin with. The piece of skin is rejected in case of too high or too low values. Due to the possibility of intra-individual variations it is important to match the pieces of skin in such a manner that creams to be compared are applied to skin from the same donor and from the same skin area. Inter-individual variations bring about that the score from different tests cannot always be compared directly.

The creams used for the skin penetration tests all contained 5% w/w acyclovir with the following propylene glycol-glycerol formal ratio:

| | Percentages by weight of glycerol formal | Percentages by weight of propylene glycol | Percentages by weight of water |
|---|---|---|---|
| 1* | 40 | 0 | 21.32 |
| 2* | 30 | 0 | 31.18 |
| 3* | 20 | 20 | 21.32 |
| 4* | 11 | 29 | 21.32 |
| 5* | 0 | 40 | 21.20 |
| 6** | 0 | 40 | 30.00 |
| 7*** | 0 | 40 | |

| | | | |
|---|---|---|---|
| *: Creams of essentially the same composition except from glycerol formal/propylene glycol, cream No. 2, however, containing additionally about 10% w/w water, the five creams all thus containing approximately 61% w/w of the aqueous phase. | | | |
| **: Composition according to DK patent No. 149.191. | | | |
| ***: Zovirax® cream | | | |

The exact composition of said seven creams mentioned appears from the following table III.

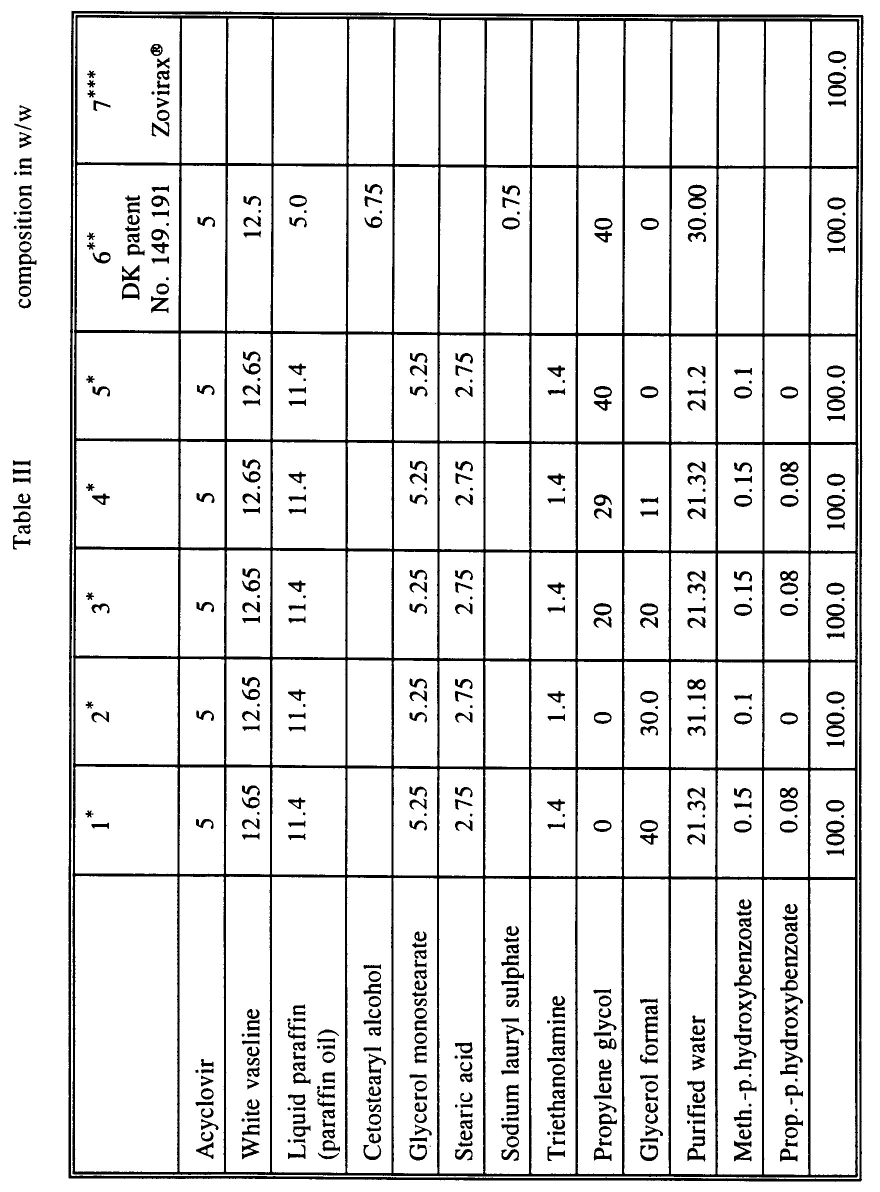

The following three tests were carried out with said seven creams:
1. Cream No. 4 was compared to cream No. 7, the amount of acyclovir dissolved in the receiver fluid being determined 24, 48, 72, 96, 120, 144 and 168 hours, respectively, subsequent to the application of cream to the piece of skin.
2. Creams No. 1, No. 3 and No. 4 were compared, the amount of acyclovir dissolved in the receiver fluid being determined 24, 48, 72, and 96 hours, respectively, subsequent to the application of cream to the piece of skin.
3. Creams No. 1, No. 2 and No. 5 were compared to cream No. 6 by measuring the amount of acyclovir dissolved in the receiver fluid 24, 48, 72, and 96 hours, respectively, subsequent to the application of cream to the piece of skin.

### Brief Description of the Drawings

The results obtained at the tests are shown on the drawings, in which
Fig. 1 shows the accumulated amount of acyclovir in the receiver fluids from cream No. 4 and cream No. 7 in the course of seven days and the distribution of the results,
Fig. 2 and 4 show the accumulated amount of acyclovir in the receiver fluids from creams No. 1, 3, and 4 in the course of four days, Fig. 4 further showing the distribution of the results (N = 5 or 4), and
Fig. 3 and 5 show the accumulated amount of acyclovir in the receiver fluids from creams No. 1, 2, 5 and 6 in the course of four days, Fig. 5 further showing the distribution of the results (N = 8).

### Best Mode for Carrying out the Invention

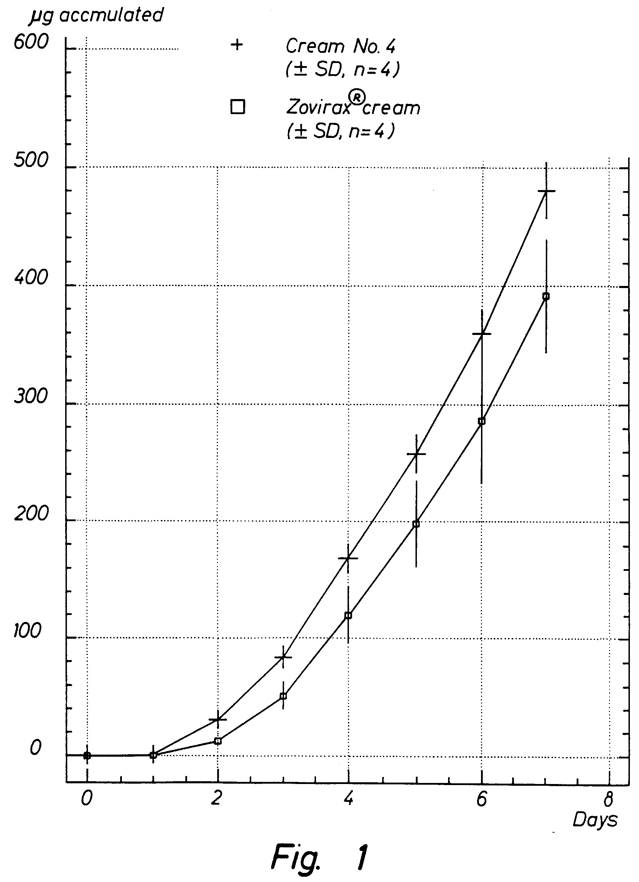

It appears from Fig. 1 that the values for the Zovirax® cream are consistently lower than the values for the cream containing 11% w/w glycerol formal and 29% w/w propylene glycol. A multifactorial analysis of variance shows that the differences are significant at all times of measurement (p less than 0.05 on day 1, 4, 5, 6 and 7; p less than 0.01 on day 2 and 3).

Test 1 thus shows that by using cream No. 4 containing 11% w/w glycerol formal and 29% w/w propylene glycol, an increased penetration of acyclovir through the skin is obtained, whereby the use of cream No. 4 results in a more rapid effect than the use of the Zovirax® cream, as a sufficient concentration of acyclovir on the site of infection is obtained more rapidly.

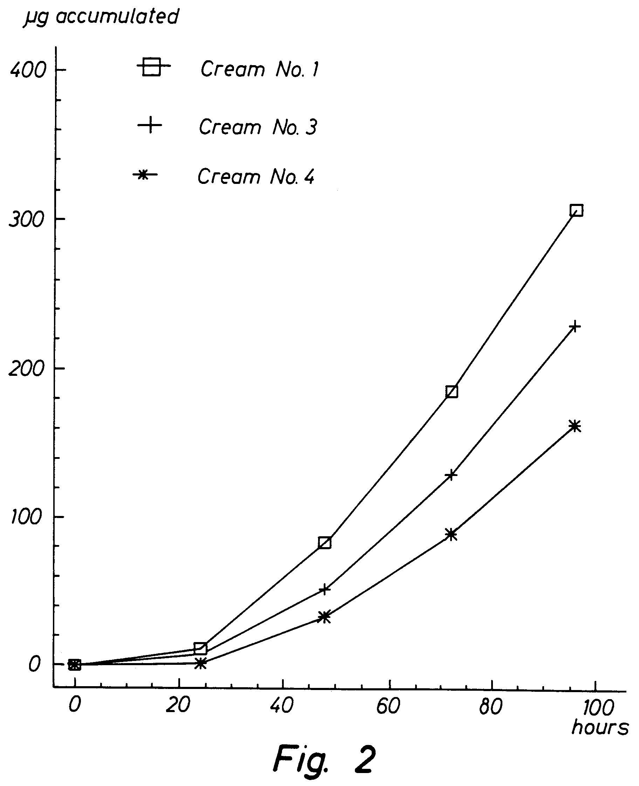

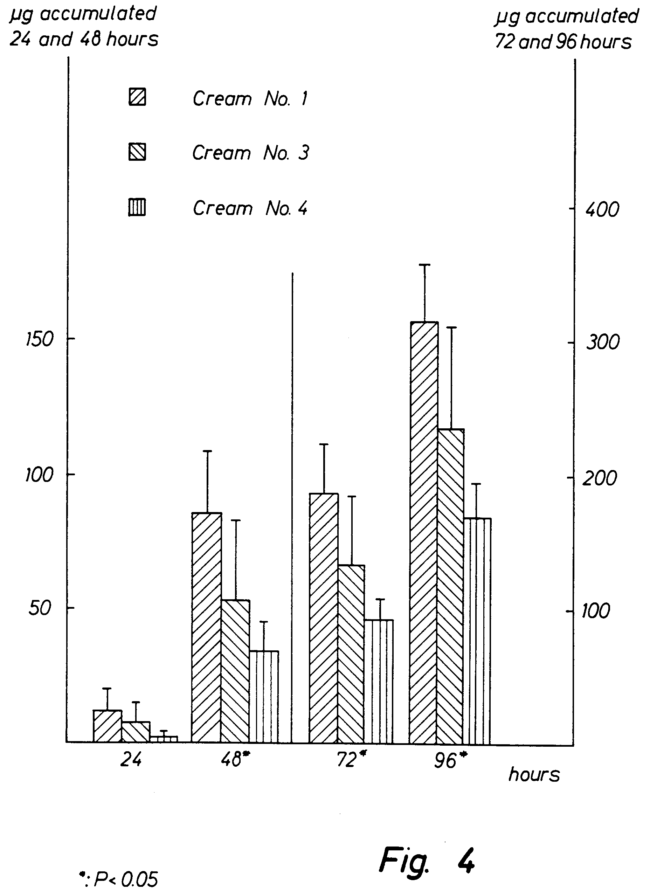

Fig. 2 and 4 show that an increasing concentration of glycerol formal from 11% w/w to 20% w/w and 40% w/w, and a concurrent reduction of the concentration of propylene glycol from 29% w/w to 20% w/w and 0% w/w result in increasing concentrations of dissolved acyclovir and thus an enhanced skin penetration of acyclovir. A multifactorial analysis of variance (p-values shown in Fig. 4) shows that the creams containing 40% w/w glycerol formal/ 0% w/w propylene glycol and 11% w/w glycerol formal/ 29% w/w propylene glycol, respectively, are significantly different as regards the penetration of acyclovir after 48, 72 and 96 hours.

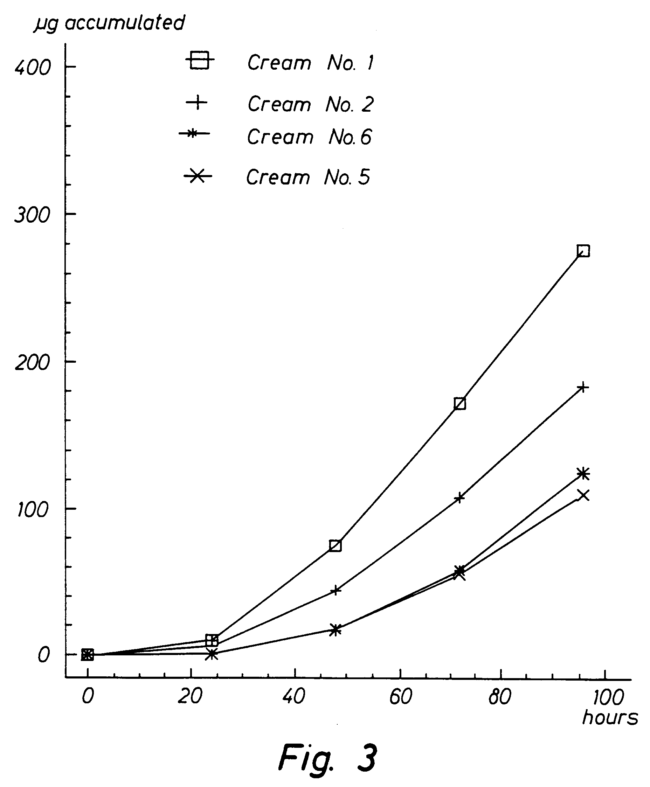

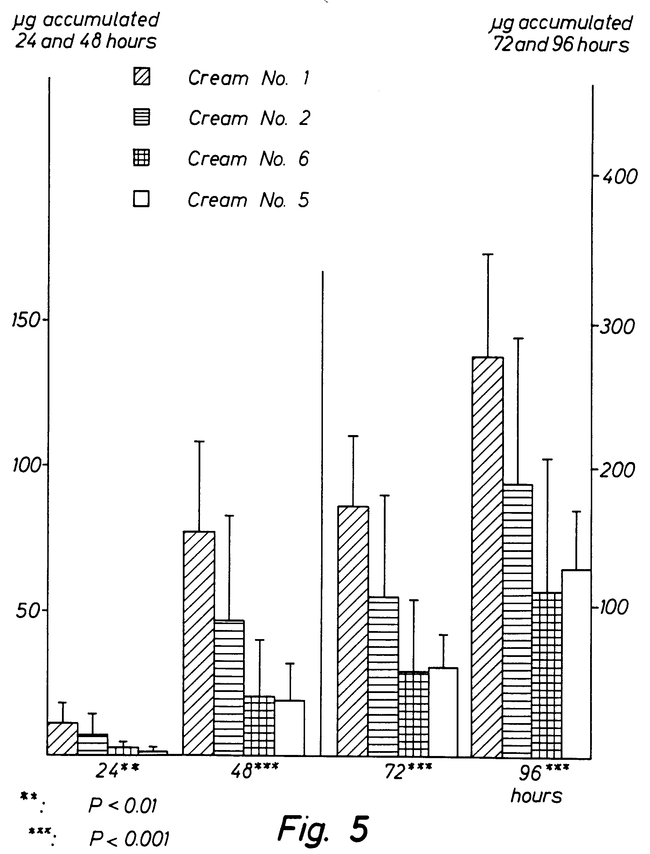

Fig. 3 and 5 show that creams containing either 40% w/w or 30% w/w glycerol formal and without any contents of propylene glycol result in an enhanced acyclovir penetration compared to the creams containing 40% w/w propylene glycol and no glycerol formal. A multifactorial analysis of variance (p-values shown in Fig. 5) shows that the cream containing 40% w/w glycerol formal is significantly different to the creams containing 40% w/w propylene glycol as regards the penetration of acyclovir after 24, 48, 72 and 96 hours.

In summary, the three tests show that the use of glycerol formal as a solvent in an oil-in-water emulsion in form of an acyclovir cream results in enhanced skin penetration of acyclovir compared to the use of propylene glycol.

This surprising effect is of great importance in the treatment of herpes (labialis), where it is important that the active medical compound reaches the site of infection rapidly and in sufficient amount.

In advantageous embodiments of the emulsion according to the invention said emulsion comprises 5% w/w of acyclovir or a salt or ester thereof and 20% w/w of glycerol formal and 20% w/w of polyhydric alcohol or 11% w/w of glycerol formal and 29% w/w of polyhydric alcohol, respectively, and from 20 to 25% w/w of water.

The polyhydric alcohol is preferably propylene glycol.

The emulsion may advantageously be in form of a cream, wherein the oil phase contains white vaseline, liquid paraffin (paraffin oil), glycerol monostearate and stearic acid.

Cream No. 4 in table III according to the invention has been examined by means animal tests and thereby compared with the Zovirax® cream containing 40% w/w propylene glycol.

### Animal test 1

The effect on cutaneous herpes virus infection in guinea pigs. HSV-1 virus was inoculated into epidermis of the back skin of 30 animals, the inoculation being made at six sites on each animal. Forty hours after the inoculation, oedema and erythema were visible on the sites of inoculation and treatment was instituted. The animals were treated twice daily for six days after the inoculation with 0.05 ml cream per site. The treatment was randomised and blinded. The treatment of animal No. 1 on the six infected sites may be illustrated as follows.
Placebo cream □ □ Acyclovir cream according to the invention
Zovirax® cream □ □ Placebo cream
Zovirax® cream □ □ Acyclovir cream according to the invention
The animal were examined daily, until all infection sores had healed completely (day 20) and the sores were rated according to their stage of development and regression by means of an arbitrary scala from 0 to 3.

At the conclusion of the test the efficacy of each treatment was estimated as:
1) Number of days to sore recovery, and
2) cumulative score of sores over the twenty days.

### Results

The number of days to recovery per treatment (geometric mean) is as follows:

| **Treatment** | **Days** | **Coefficient of variance** |
|---|---|---|
| Acyclovir cream according to the invention | 6.9 | 0.38 |
| Zovirax® cream | 8.5 | 0.31 |
| Placebo cream | 10.5 | 0.21 |

A statistically significant difference between the three treatments (p < 0.00001) was found. The sites treated with the acyclovir cream according to the invention showed the fastest regression of sores followed by the sites treated with the Zovirax® cream, which were followed by the sites treated with the placebo cream.

The cumulative score of sores over the twenty days (mean values) is as follows:

| **Treatment** | **Score** | **95% confidence for limit** |
|---|---|---|
| Acyclovir cream according to the invention | 7.1 | 6.09 to 8.12 |
| Zovirax® cream | 9.0 | 8.02 to 10.0 |
| Placebo cream | 16.3 | 15.29 to 17.25 |

There was a statistically significant difference (p < 0.00001) between the three cream for treatment. The acyclovir cream according to the invention gave the lowest cumulative score followed by the Zovirax® cream and then the placebo cream.

The conclusion as regards the result of the study of the effect of creams on cutaneous herpes virus infection in guinea pigs is that the study clearly demonstrates the effect of acyclovir creams on cutaneous herpes virus infections in guinea pigs. With reference to the above skin penetration tests, the study further showed a correlation between the in vitro test results with human skin and in vivo test results with guinea pigs, the acyclovir cream according to the invention showing a more rapid skin penetration in vitro and a better treatment effect in vivo compared to the Zovirax® cream.

### Animal test 2

A fourteen-day cumulative skin irritation study in rabbits. Six rabbits were treated for 14 days with the acyclovir cream according to the invention, the Zovirax® cream and a 2% carbamide cream on the shaven backs. The dose was 0.05 ml per treatment site per day. Skin erythema was scored arbitrarily once a day by means of the following scoring system. The skin thickness was also measured daily with a thickness meter.

| **Formation of erythema and crust of a sore** | **Value** |
|---|---|
| No erythema | 0 |
| Very slight erythema (barely perceptible) | 1 |
| Well-defined erythema | 2 |
| Moderate to severe erythema | 3 |
| Severe erythema (beet redness) to slight formation of crust (lesions in depth) | 4 |

Scaling without erythema was set to a score of 1.

### Results

A cumulative index per treatment was calculated regarding erythema. The maximum obtainable index was 40. The results appear from the following table IV.

Scaly skin was observed on three occasions in the sites treated with the Zovirax® cream.

The treatment with the Zovirax® cream resulted in a significantly higher skin irritation index compared to the treatment with the acyclovir cream according to the invention and the 2% carbamide cream (p = 0.0002).

The skin irritation index per treatment as regards change in the skin thickness (primarily a measure for fluid accumulation) was calculated. No maximum obtainable index can be deduced from this measurement. The results are shown in the following table IV. No significant differences were found regarding changes in the skin thickness (p > 0.05).

**Table IV**

| Skin irritation index regarding erythema and change in the skin thickness. | | |
|---|---|---|
| **Preparation** | **Erythema** | **Change in skin thickness** |
| 5% Acyclovir cream according to the invention | 15.0 | 0.148 |
| 5% Zovirax® cream | 23.2 | 0.198 |
| 2% Carbamide cream | 12.5 | 0.157 |

The study of skin irritation at the use of the above acyclovir-containing creams shows that the cream according to the invention is significantly less irritating to rabbit skin compared to the Zovirax® cream. However, both creams cause a clearly visible skin erythema as did the carbamide cream used as a reference, the carbamide cream being a commonly used emollient. The highest score for erythema obtained in the study was 3, which corresponds to moderate to severe erythema. As rabbit skin is approximately four times as permeable as human skin, but otherwise comparable to covered human skin, the severe skin changes are not liable to occur in human beings.

An acyclovir cream according to the invention may be prepared in a simple manner by dissolving the substances (preservatives) stated under I in the following table V in purified water (II) during heating to 80°C, whereafter the organic solvent comprising glycerol formal and possibly propylene glycol and triethanolamine (III) is admixed. The acyclovir (V) is then dispersed in the mixture and the temperature is set to between 57°C and 63°C. The substances stated under IV are melted together and admixed at said temperature, whereafter emulsification is carried out to obtain a durable oil-in-water emulsion. The finished emulsion is cooled down to room temperature while being stirred continuously.

**Table V**

| Cream compositions in % w/w | | | | | |
|---|---|---|---|---|---|
| Acyclovir | 5 | 5 | 5 | 5 | V |
| White vaseline | 12.65 | 12.65 | 12.65 | 15 | IV |
| Liquid paraffin (paraffin oil) | 11.4 | 11.4 | 11.4 | 6 | |
| Cetomacrogol 1.000 | | | | 1.8 | |
| Cetostearyl alcohol | | | | 7.2 | |
| Glycerol monostearate | 5.25 | 5.25 | 5.25 | | |
| Stearic acid | 2.75 | 2.75 | 2.75 | | |
| Triethanolamine | 1.4 | 1.4 | 1.4 | | III |
| Propylene glycol | 29 | 20 | 0 | | |
| Glycerol formal | 11 | 20 | 40 | 40 | |
| Purified water | 21.32 | 21.32 | 21.32 | 24.77 | II |
| Meth.-p.hydroxybenzoate | 0.15 | 0.15 | 0.15 | 0.15 | I |
| Prop.-p.hydroxybenzoate | 0.08 | 0.08 | 0.08 | 0.08 | |
| | 100.0g | 100.0g | 100.0g | 100.0g | |

## Claims

1. An antivirally active pharmaceutical oil-in-water emulsion containing 9-[(2-hydroxyethoxy)methyl]guanine (acyclovir) or a salt or ester thereof as an active ingredient in the continuous aqueous phase, said phase in addition to said active ingredient and the dispersed oil phase containing a water miscible organic solvent, wherein a polyhydric alcohol may form a constituent, **characterised** in that the emulsion comprises from 1% to 10% w/w of acyclovir or a salt or ester thereof, from 20% to 50 % w/w of organic solvent comprising from 5% to 50% w/w glycerol formal and from 0% to 29% w/w of a polyhydric alcohol, and from 20% to 40 % w/w water, said percentages being based on the total weight of the formulation.

2. An emulsion as claimed in claim 1, **characterised** in that it comprises from 2% to 5% w/w of acyclovir or a salt or ester thereof, from 30% to 45% w/w of solvent, and from 15% to 35% w/w water.

3. An emulsion as claimed in claim 2, **characterised** in that it comprises 5% w/w acyclovir or a salt or ester thereof, 40% w/w glycerol formal, and from 15% to 30% w/w water.

4. An emulsion as claimed in claim 2, **characterised** in that it comprises 5% w/w acyclovir or a salt or ester thereof, 20% w/w glycerol formal, 20% w/w polyhydric alcohol, and from 20% to 25% w/w water.

5. An emulsion as claimed in claim 2, **characterised** in that it comprises 5% w/w acyclovir or a salt or ester thereof, 11% w/w glycerol formal, 29% w/w polyhydric alcohol, and from 20% to 25% w/w water.

6. An emulsion as claimed in any of the preceding claims, **characterised** in that the polyhydric alcohol is propylene glycol.

7. An emulsion as claimed in any of the preceding claims, **characterised** in that it is available as a cream, wherein the oil phase comprises white vaseline, liquid paraffin (paraffin oil), glycerol monostearate, and stearic acid.

## Patentansprüche

1. Antiviral wirkende pharmazeutische Öl-in-Wasser-Emulsion, die 9-[(2-hydroxyethoxy)methyl]guanin (Acyclovir) oder ein Salz oder einen Ester davon als einen Wirkstoff in der kontinuierlichen wässrigen Phase enthält, wobei die Phase außer dem Wirkstoff und der dispersen Ölphase ein mit Wasser mischbares organisches Lösungsmittel enthält, bei dem ein mehrwertiger Alkohol einen Bestandteil bilden kann, dadurch gekennzeichnet, daß die Emulsion 1 % bis 10 % w/w Acyclovir oder ein Salz oder einen Ester davon, 20 % bis 50 % w/w eines organischen Lösungsmittels, das 5 % bis 50 % w/w Glycerinformal und 0 % bis 29 % w/w eines mehrwertigen Alkohols enthält, und 20 % bis 40 % w/w Wasser aufweist, wobei die Prozentsätze auf das Gesamtgewicht der Formulierung bezogen sind.

2. Emulsion nach Anspruch 1, dadurch gekennzeichnet, daß sie 2 % bis 5 % w/w Acyclovir oder ein Salz oder einen Ester davon, 30 % bis 45 % w/w eines Lösungsmittels und 15 % bis 35 % w/w Wasser aufweist.

3. Emulsion nach Anspruch 2, dadurch gekennzeichnet, daß sie 5 % w/w Acyclovir oder ein Salz oder einen Ester davon, 40 % w/w Glycerinformal und 15 % bis 30 % w/w Wasser aufweist.

4. Emulsion nach Anspruch 2, dadurch gekennzeichnet, daß sie 5 % w/w Acyclovir oder ein Salz oder einen Ester davon, 20 % w/w Glycerinformal, 20 % w/w mehrwertigen Alkohol und 20 % bis 25 % w/w Wasser aufweist.

5. Emulsion nach Anspruch 2, dadurch gekennzeichnet, daß sie 5 % w/w Acyclovir oder ein Salz oder einen Ester davon, 11 % w/w Glycerinformal, 29 % w/w mehrwertigen Alkohol und 20 % bis 25 % w/w Wasser aufweist.

6. Emulsion nach einem der vorhergehenden Ansprüche, dadurch gekennzeichnet, daß der mehrwertige Alkohol Propylenglycol ist.

7. Emulsion nach einem der vorhergehenden Ansprüche, dadurch gekennzeichnet, daß sie als eine Creme erhältlich ist, bei der die Ölphase weiße Vaseline, flüssiges Paraffin (Paraffinöl), Glycerinmonostearat und Sterinsäure aufweist.

## Revendications

1. Emulsion pharmaceutique huile dans l'eau à activité antivirale contenant la 9-[(2-hydroxyéthoxy)méthyl]guanine (acyclovir) ou un sel ou ester de celle-ci en tant que principe actif dans la phase aqueuse continue, ladite phase, en plus dudit principe actif, et la phase huileuse dispersée contenant un solvant organique miscible à l'eau, dans lequel un polyol peut former un constituant, caractérisée en ce que l'émulsion comprend de 1% à 10% en poids d'acyclovir ou d'un sel ou ester de celui-ci, de 20% à 50% en poids de solvant organique comprenant de 5% à 50% en poids de glycérol formal et de 0% à 29% en poids d'un polyol, et de 20% à 40% en poids d'eau, lesdits pourcentages étant par rapport au poids total de la formulation.

2. Emulsion selon la revendication 1, caractérisée en ce qu'elle comprend de 2% à 5% en poids d'acyclovir ou d'un sel ou ester de celui-ci, de 30% à 45% en poids de solvant et de 15% à 35% en poids d'eau.

3. Emulsion selon la revendication 2, caractérisée en ce qu'elle comprend 5% en poids d'acyclovir ou d'un sel ou ester de celui-ci, 40% en poids de glycérol formal et de 15% à 30% d'eau.

4. Emulsion selon la revendication 2, caractérisée en ce qu'elle comprend 5% en poids d'acyclovir ou d'un sel ou ester de celui-ci, 20% en poids de glycérol formal, 20% en poids de polyol et de 20% à 25% en poids d'eau.

5. Emulsion selon la revendication 2, caractérisée en ce qu'elle comprend 5% en poids d'acyclovir ou d'un sel ou ester de celui-ci, 11% en poids de glycérol formal, 29% en poids de polyol et de 20% à 25% en poids d'eau.

6. Emulsion selon l'une quelconque des revendications précédentes, caractérisée en ce que le polyol est le propylèneglycol.

7. Emulsion selon l'une quelconque des revendications précédentes, caractérisée en ce qu'elle est disponible sous forme de crème, dans laquelle la phase huileuse comprend de la vaseline blanche, de la paraffine liquide (huile de paraffine), du monostéarate de glycérol et de l'acide stéarique.
